# FASCICULE DE BREVET EUROPEEN

(11) **EP 3 471 893 B1**
(45) Date de publication et mention de la délivrance du brevet: **05.08.2020**
(21) Numéro de dépôt: 17734788.7
(22) Date de dépôt: 08.06.2017
(51) Int. Cl.: B05B 11/02, B05B 11/06, A61M 11/02, A61M 15/00

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 20.06.2016 FR 1655706
(43) Date de publication de la demande: 24.04.2019
(73) Titulaire: Aptar France SAS, 27110 Le Neubourg (FR)
(72) Inventeur: POULLAIN, Franck, 27400 La Haye-Malherbe (FR)
(74) Mandataire: CAPRI
(86) Numéro de dépôt international: PCT/FR2017/051441
(87) Numéro de publication internationale: WO 2017/220879

(56) Documents cités:
- WO-A1-02/45866
- WO-A1-2014/147329
- FR-A1- 3 007 992

## Description

La présente invention concerne un dispositif de distribution de produit fluide ou pulvérulent, et plus particulièrement un dispositif pour distribuer unitairement une seule dose de produit contenue dans un réservoir à l'aide d'un écoulement d'air sous pression.

Les documents WO9946055 et WO0245866 divulguent des dispositifs de ce type. Un inconvénient de ces dispositifs est qu'il peut être difficile, notamment pour un utilisateur novice, de savoir si le dispositif a ou non été utilisé. Il peut alors se retrouver avec un dispositif en pensant qu'il contient une dose à distribuer alors qu'en réalité il est vide.

Les documents FR3007992 et WO2014147329 décrivent d'autres dispositifs de l'état de la technique.

La présente invention a pour but de fournir un dispositif de distribution de produit fluide ou pulvérulent qui ne reproduit pas les inconvénients susmentionnés.

La présente invention a aussi pour but de fournir un tel dispositif de distribution de produit fluide ou pulvérulent qui donne une information claire à l'utilisateur pour lui permettre de savoir si le dispositif a ou non été actionné.

La présente invention a aussi pour but de fournir un dispositif de distribution de produit fluide ou pulvérulent, qui soit simple et peu coûteux à fabriquer et à assembler.

La présente a donc pour objet un dispositif de distribution de produit fluide ou pulvérulent comportant une tête de distribution pourvue d'une sortie de distribution formée à l'extrémité d'un manchon creux, une chasse d'air pour générer un écoulement d'air lors de l'actionnement du dispositif, ladite chasse d'air comportant un piston coulissant dans une chambre d'air entre une position de repos et une position de distribution, ladite chambre d'air comportant un corps cylindrique dans lequel ledit piston coulisse de manière étanche, et un réservoir contenant une dose unique de produit, ledit réservoir comportant une entrée d'air reliée à ladite chasse d'air et une sortie de produit reliée à ladite sortie de distribution, ladite entrée d'air comportant un organe de retenue de produit pour maintenir le produit dans le réservoir jusqu'à la distribution du produit, et ladite sortie de produit étant obturée par un élément de fermeture emmanché à force dans la sortie de produit du réservoir, ledit dispositif comportant un système d'ouverture mécanique coopérant avec ledit élément de fermeture pour l'expulser mécaniquement de sa position d'obturation lors de l'actionnement du dispositif, ledit dispositif comportant un indicateur qui en position de repos avant actionnement est dans un premier état, et qui après actionnement passe dans un second état, ce passage du premier au second état informant l'utilisateur que le dispositif a été actionné, ledit premier état étant un état visible et ledit second état étant un état invisible, ledit dispositif comportant un élément poussoir, solidaire dudit piston, sur lequel l'utilisateur appuie lors de l'actionnement, ladite tête de distribution comportant une jupe disposée autour de ladite chambre d'air, le bord axial inférieur de ladite jupe comportant une découpe.

Avantageusement, ledit indicateur est formé par un organe souple coulissant, solidaire d'un côté dudit élément poussoir et dont le côté opposé forme une extrémité libre qui, en position de repos avant actionnement, s'étend entre l'extérieur de la ladite chambre d'air et l'intérieur de ladite jupe, en étant visible à travers ladite découpe.

Avantageusement, lors de l'actionnement, ledit élément poussoir est déplacé axialement par rapport à ladite jupe en tirant sur ledit indicateur (100), de sorte qu'en fin d'actionnement, ledit indicateur est disposé en totalité à l'intérieur de ladite chambre d'air.

Ces caractéristiques et avantages et d'autres apparaîtront plus clairement au cours de la description détaillée suivante d'un mode de réalisation, faite en référence aux dessins joints, donnés à titre d'exemples non limitatifs, et sur lesquels :
- la figure 1a est une vue schématique de côté d'un dispositif de distribution de produit fluide ou pulvérulent selon un premier mode de réalisation, en position de repos avant actionnement,
- la figure 1b est une vue schématique en section transversale similaire à celle de la figure 1a,
- la figure 2a est une vue schématique de côté du dispositif de la figure 1a, après actionnement, et
- la figure 2b est une vue schématique en section transversale similaire à celle de la figure 2a.

La présente invention concerne plus particulièrement un dispositif du type de celui divulgué dans le document WO0245866. Il est toutefois entendu que la présente invention ne se limite pas à ce type de dispositif, mais est au contraire applicable à tous les types de dispositifs de distribution de produit fluide et pulvérulents du type unidose, c'est-à-dire comportant un réservoir ne contenant qu'une seule dose distribuée en un seul actionnement.

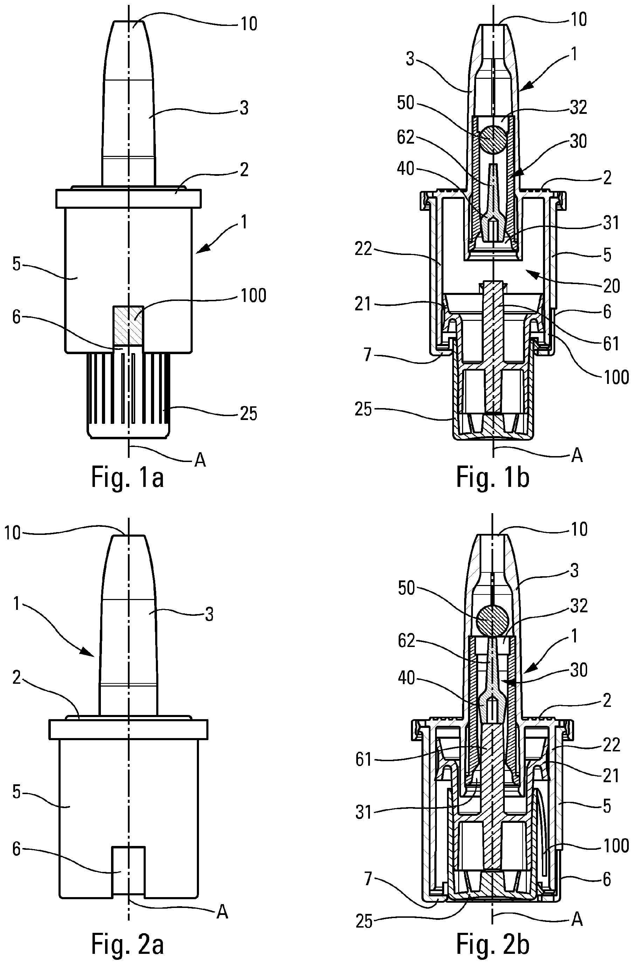

Dans la description ci-après, les termes "supérieur", "inférieur", "haut" et "bas" se réfèrent à la position droite du dispositif représentée sur les figures. Les termes "axial" et "radial" se réfèrent à l'axe central longitudinal A du dispositif, visible sur les figures 1a, 1b, 2a et 2b.

Le dispositif comporte un réservoir 30 comportant une entrée d'air 31 et une sortie de produit 32, et contenant une dose unique de produit fluide, liquide ou pulvérulent. L'entrée d'air 31 du réservoir est reliée à une chasse d'air 20 et la sortie de produit 32 du réservoir est reliée à une sortie de distribution 10 du dispositif. La sortie de produit 32 est obturée par un élément de fermeture 50 qui est emmanché à force dans ladite sortie de produit 32. L'entrée d'air 31 est pourvue d'un organe de retenue de produit 40 qui est adapté à maintenir le produit dans le réservoir 30 avant l'actionnement du dispositif. La chasse d'air 20 est actionnée manuellement par l'utilisateur et est adaptée à créer un écoulement d'air qui va traverser le réservoir 30 pour emmener le produit qu'il contient en direction de la sortie de distribution 10.

Le réservoir 30 est solidaire, notamment emmanché, dans une tête de distribution 1 qui comporte la sortie de distribution 10. La tête de distribution 1 comporte avantageusement un repose-doigt 2 s'étendant radialement pour faciliter l'actionnement. Un manchon creux 3 s'étend axialement vers le haut à partir dudit repose-doigt 2 et se termine au niveau de ladite sortie de distribution 10. De préférence, ce manchon creux 3 est de dimension radiale réduite pour pouvoir être inséré dans une narine au moment de l'actionnement. Du côté opposé du repose-doigt 2, la tête de distribution 1 comporte une jupe 5 s'étendant axialement vers le bas à partir dudit repose-doigt 2.

Le dispositif comporte un système d'ouverture mécanique 61, 62, qui est de préférence solidaire de la chasse d'air 20, c'est à dire qu'il est actionné simultanément à l'actionnement de ladite chasse d'air 20, et qui est adapté à coopérer avec ledit élément de fermeture 50 pour l'expulser mécaniquement de sa position d'obturation lors de l'actionnement du dispositif. Dans l'exemple représenté sur les figures, le système d'ouverture mécanique comporte un ensemble de tiges 61, 62, dont une première partie de tige 61 est solidaire de la chasse d'air 20, et une seconde partie de tige 62 est poussée par ladite première partie de tige 61 lorsque le dispositif est actionné. L'ensemble de tiges 61, 62 va en fin de course d'actionnement, c'est à dire en position de distribution, coopérer avec l'élément de fermeture 50 pour l'expulser mécaniquement de sa position d'obturation.

L'organe de retenue de produit 40 peut être avantageusement réalisé monobloc avec la seconde partie de tige 62. Ainsi, l'organe de retenue de produit 40 peut être réalisé de manière étanche au produit et étanche à l'air, avant l'actionnement du dispositif, la pression d'air créée par la chasse d'air 20 ne pénétrant à l'intérieur du réservoir 30 qu'au moment où ledit organe de retenu 40 est déplacé ensemble avec la seconde partie de tige 62, en étant poussé par la première partie de tige 61.

L'élément de fermeture 50 peut être sphérique, par exemple une bille plastique, comme représenté sur les figures 1b et 2b.

La chasse d'air 20, représentée sur les figures 1b et 2b, comporte un piston 21 coulissant dans une chambre d'air 22, le piston 21 étant actionné manuellement par l'utilisateur. La chambre d'air 22 peut être formée par un cylindre axial creux monobloc avec le repose-doigt 2 de la tête de distribution 1, le côté inférieur de ladite chambre d'air 22 étant ouvert et obturé par ledit piston 21. La jupe 5 de la tête est alors avantageusement disposée autour de ladite chambre d'air 22, et peut notamment être formée par un cylindre creux fixé, par exemple encliqueté, dans le repose-doigt 2 de la tête de distribution 1.

Avantageusement, pour protéger le piston 21, le bord inférieur de la jupe 5 entoure le bord inférieur de la chambre d'air 22, et comporte une bride radiale 7 s'étendant radialement vers l'intérieur sous ledit bord inférieur de la chambre d'air 22.

Avantageusement, l'actionnement du dispositif est réalisé au moyen d'un élément poussoir 25 assemblé sur ledit piston 21.

Le piston 21 est solidaire de la première partie de tige 61, avantageusement en étant formé sur une même pièce monobloc.

Lorsque l'utilisateur souhaite actionner le dispositif, il place d'une part ses doigts sur le repose-doigt 2 de la tête de distribution 1 et d'autre part son pouce sur l'élément poussoir 25, et il exerce une force axiale d'actionnement, qui va déplacer la première partie de tige 61 et le piston 21 vers la position de distribution. Le piston 21 de la chasse d'air coopère de manière étanche avec la chambre d'air 22, de sorte que l'air contenu dans ladite chambre d'air 22 va être progressivement comprimé au cours de l'actionnement.

Après une course d'actionnement initiale de compression d'air, l'extrémité axiale supérieure de la première partie de tige 61 vient en contact de l'organe de retenue 40 et donc de la seconde partie de tige 62.

Une poursuite de l'actionnement déplace ledit organe de retenue 40 axialement vers le haut dans le réservoir 30, donc hors de sa position d'obturation ou de fermeture étanche de l'entrée d'air 32. A ce moment-là, l'air comprimé dans la chambre d'air 22 peut donc pénétrer dans le réservoir 30. Au même moment, l'extrémité axiale supérieure de la seconde partie de tige 62 vient en contact de l'élément de fermeture 50.

Une poursuite de l'actionnement va donc déplacer l'élément de fermeture 50 axialement vers le haut, hors de sa position d'obturation.

Lorsque l'étanchéité de l'élément de fermeture 50 est rompue, celui-ci est expulsé hors du réservoir 30 pour permettre la distribution du produit fluide ou pulvérulent sous l'effet de l'air comprimé. L'élément de fermeture 50 vient alors se coincer dans des nervures de la tête de distribution 1, qui empêchent notamment tout risque d'expulsion dudit élément de fermeture hors de ladite tête de distribution 1. La position de distribution, en fin de course d'actionnement et après distribution du produit, est représentée sur les figures 2a et 2b.

Selon l'invention, le dispositif comporte un indicateur qui permet d'informer l'utilisateur sur l'état du dispositif, à savoir avant ou après actionnement. Cet indicateur passe d'un premier état en position de repos avant actionnement à un second état après actionnement, ce passage du premier au second état informant l'utilisateur que le dispositif a été actionné.

Selon l'invention, l'indicateur est clairement visible avant actionnement et disparait après actionnement.

Les figures 1a,b et 2a,b illustrent un mode de réalisation avantageux.

Selon l'invention, la jupe 5 de la tête de distribution 1 comporte une découpe 6 au niveau de son bord axial inférieur. Un indicateur 100 est visible à travers cette découpe 6 avant actionnement et n'est plus visible après actionnement.

Cet indicateur 100 est avantageusement formé par un organe souple coulissant solidaire d'un côté de l'élément poussoir 25 et dont le côté opposé forme une extrémité libre. Ainsi, en position de repos avant actionnement, l'extrémité libre de cet indicateur 100 s'étend autour du bord inférieur de la chambre d'air 22 et entre l'extérieur de la ladite chambre d'air 22 et l'intérieur de ladite jupe 5. L'indicateur 100 est de ce fait visible à travers la découpe 6 de la jupe 5.

Lorsque le dispositif est actionné, l'élément poussoir 25 est déplacé axialement vers le haut par rapport à la jupe 5. Lors de ce déplacement axial, l'indicateur 100 est tiré par ledit élément poussoir 25 de sorte qu'il est progressivement retiré de la découpe 6. En fin d'actionnement, l'indicateur 100 est en totalité disposé à l'intérieur de la chambre d'air 22, comme visible sur la figure 2b, et donc n'est plus visible à travers la découpe 6 de la jupe 5.

D'autres mises en oeuvre sont possibles pour l'indicateur.

La présente invention a été décrite en référence à un mode de réalisation, mais il est entendu qu'un homme du métier peut y apporter toutes modifications, sans sortir du cadre de la présente invention tel que défini par les revendications annexées.

## Revendications

1. Dispositif de distribution de produit fluide ou pulvérulent comportant une tête de distribution (1) pourvue d'une sortie de distribution (10) formée à l'extrémité d'un manchon creux (3), une chasse d'air (20) pour générer un écoulement d'air lors de l'actionnement du dispositif, ladite chasse d'air comportant un piston (21) coulissant dans une chambre d'air (22) entre une position de repos et une position de distribution, ladite chambre d'air (22) comportant un corps cylindrique dans lequel ledit piston (21) coulisse de manière étanche, et un réservoir (30) contenant une dose unique de produit, ledit réservoir (30) comportant une entrée d'air (31) reliée à ladite chasse d'air (20) et une sortie de produit (32) reliée à ladite sortie de distribution (10), ladite entrée d'air (31) comportant un organe de retenue de produit (40) pour maintenir le produit dans le réservoir (30) jusqu'à la distribution du produit, et ladite sortie de produit (32) étant obturée par un élément de fermeture (50) emmanché à force dans la sortie de produit (32) du réservoir (30), ledit dispositif comportant un système d'ouverture mécanique (61, 62) coopérant avec ledit élément de fermeture (50) pour l'expulser mécaniquement de sa position d'obturation lors de l'actionnement du dispositif, ledit dispositif comportant un indicateur (100) qui en position de repos avant actionnement est dans un premier état, et qui après actionnement passe dans un second état, ce passage du premier au second état informant l'utilisateur que le dispositif a été actionné, ledit premier état étant un état visible et ledit second état étant un état invisible, ledit dispositif comportant un élément poussoir (25), solidaire dudit piston (21), sur lequel l'utilisateur appuie lors de l'actionnement, ladite tête de distribution (1) comportant une jupe (5) disposée autour de ladite chambre d'air (22), **caractérisé en ce que** le bord axial inférieur de ladite jupe (5) comporte une découpe (6).

2. Dispositif selon la revendication 1, dans lequel ledit indicateur (100) est formé par un organe souple coulissant, solidaire d'un côté dudit élément poussoir (25) et dont le côté opposé forme une extrémité libre qui, en position de repos avant actionnement, s'étend entre l'extérieur de la ladite chambre d'air (22) et l'intérieur de ladite jupe (5), en étant visible à travers ladite découpe (6).

3. Dispositif selon la revendication 2, dans lequel, lors de l'actionnement, ledit élément poussoir (25) est déplacé axialement par rapport à ladite jupe (5) en tirant sur ledit indicateur (100), de sorte qu'en fin d'actionnement, ledit indicateur (100) est disposé en totalité à l'intérieur de ladite chambre d'air (22).

## Patentansprüche

1. Vorrichtung zur Ausgabe eines Fluid- oder Pulverprodukts, umfassend einen Spenderkopf (1), der mit einer Abgabeöffnung (10) versehen ist, die am Ende einer hohlen Hülse (3) ausgebildet ist, eine Luftstoßeinrichtung (20) zum Erzeugen eines Luftstroms bei Betätigung der Vorrichtung, wobei die Luftstoßeinrichtung einen Kolben (21) umfasst, der in einer Luftkammer (22) zwischen einer Ruheposition und einer Abgabeposition gleitet, wobei die Luftkammer (22) einen zylindrischen Körper, in dem der Kolben (21) dicht gleitet, und einen Behälter (30), der eine Einzeldosis des Produkts enthält, umfasst, wobei der Behälter (30) einen mit der Luftstoßeinrichtung (20) verbundenen Lufteinlass (31) und einen mit der Abgabeöffnung (10) verbundenen Produktauslass (32) umfasst, wobei der Lufteinlass (31) ein Produktrückhalteorgan (40) aufweist, um das Produkt bis zur Abgabe des Produkts in dem Behälter (30) zu halten, und der Produktauslass (32) durch ein Verschlusselement (50) verschlossen ist, das in den Produktauslass (32) des Behälters (30) eingepresst ist, wobei die Vorrichtung ein mechanisches Öffnungssystem (61, 62) aufweist, mit dem Verschlusselement (50) zusammenwirkt, um es bei Betätigung der Vorrichtung mechanisch aus seiner Verschlussposition herauszutreiben, wobei die Vorrichtung eine Anzeige (100) aufweist, die sich in der Ruhestellung vor der Betätigung in einem ersten Zustand befindet und die nach der Betätigung in einen zweiten Zustand übergeht, wobei dieser Übergang von dem ersten in den zweiten Zustand den Benutzer darüber informiert, dass die Vorrichtung betätigt wurde, wobei der erste Zustand ein sichtbarer Zustand und der zweite Zustand ein unsichtbarer Zustand ist, wobei die Vorrichtung ein fest mit dem Kolben (21) verbundenes Drückerelement (25) aufweist, auf das der Benutzer bei der Betätigung drückt, wobei der Spenderkopf (1) eine Ummantelung (5) aufweist, die um die Luftkammer (22) herum angeordnet ist, **dadurch gekennzeichnet, dass** der untere axiale Rand der Ummantelung (5) einen Ausschnitt (6) umfasst.

2. Vorrichtung nach Anspruch 1, bei der die Anzeige (100) durch ein flexibles Schiebeelement gebildet wird, das an einer Seite fest mit dem Drückerelement (25) verbunden ist und dessen gegenüberliegende Seite ein freies Ende bildet, das sich in der Ruhestellung vor der Betätigung zwischen der Außenseite der Luftkammer (22) und der Innenseite der Ummantelung (5) erstreckt, wobei es in dem Ausschnitt (6) sichtbar ist.

3. Vorrichtung nach Anspruch 2, bei der bei deren Betätigung das Drückerelement (25) durch Ziehen an der Anzeige (100) bezüglich der Ummantelung (5) axial verschoben wird, so dass die Anzeige (100) am Ende der Betätigung vollständig im Inneren der Luftkammer (22) angeordnet ist.

## Claims

1. A dispenser device for dispensing a fluid or powder composition, the dispenser device comprising: a dispenser head (1) that is provided with a dispenser outlet (10) that is formed at the end of a hollow sleeve (3), an air expeller (20) for generating a flow of air while the device is being actuated, said air expeller including a piston (21) that slides in an air chamber (22) between a rest position and a dispensing position, said air chamber (22) including a cylindrical body in which said piston (21) slides in airtight manner, and a reservoir (30) that contains a single dose of composition, said reservoir (30) including an air inlet (31) that is connected to said air expeller (20), and a composition outlet (32) that is connected to said dispenser outlet (10), said air inlet (31) including a composition retainer member (40) for retaining the composition in the reservoir (30) until the composition is dispensed, and said composition outlet (32) being closed by a closure element (50) that is force fitted in the composition outlet (32) of the reservoir (30), said device further comprising a mechanical opening system (61, 62) co-operating with said closure element (50) so as to expel it mechanically from its closed position while the device is being actuated, said device comprising an indicator (100) that, in the rest position before actuation is in a first state, and that after actuation passes into a second state, this passage from the first to the second state informing the user that the device has been actuated, said first state being a visible state and said second state being a non-visible state, said device including a pusher element (25), that is secured to said piston (21), and on which the user presses during actuation, said dispenser head (1) including a skirt (5) arranged around said air chamber (22), **characterized in that** the bottom axial edge of said skirt (5) includes a cutout (6).

2. A device according to claim 1, wherein said indicator (100) is formed by a slidable flexible member, secured at one end to said pusher element (25), and having an opposite end that forms a free end that, in the rest position before actuation, extends between the outside of said air chamber (22) and the inside of said skirt (5), being visible through said cutout (6).

3. A device according to claim 2, wherein, during actuation, said pusher element (25) is moved axially relative to said skirt (5), pulling on said indicator (100), so that at the end of actuation, all of said indicator (100) is arranged inside said air chamber (22).
